# EUROPEAN PATENT SPECIFICATION

(11) **EP 3 190 139 B1**
(45) Date of publication and mention of the grant of the patent: **28.08.2019**
(21) Application number: 17150788.2
(22) Date of filing: 10.01.2017
(51) Int. Cl.: C08G 75/0204, C07C 315/00, C07C 317/22, C07C 323/00, C08J 5/00, C08G 75/025

(54) **METHOD FOR PREPARING AN AROMATIC SULFIDE OR SALT THEREOF**
VERFAHREN ZUR HERSTELLUNG VON POLYARYLENSULFID (PAS)-MONOMER
PROCÉDÉ DE PRÉPARATION DE SULFURE DE POLYARYLÈNE (PA) MONOMÈRE

(30) Priority: 11.01.2016 US 201662277091 P; 20.12.2016 TW 105142207
(43) Date of publication of application: 12.07.2017
(73) Proprietor: Industrial Technology Research Institute, Chutung, Hsinchu 31040 (TW)
(72) Inventor: Ho, Po-Hsien, 112 Taipei City (TW); Lin, Chih-Hsiang, 105 Taipei City (TW); Chen, Meng-Hsin, 925 Pingtung County (TW); Fan, Cheng-Hsing, 709 Tainan City (TW); Kao, Hsin-Ching, 308 Hsinchu County (TW); Chang, Yih-Her, 308 Hsin Chu County (TW)
(74) Representative: Michalski Hüttermann & Partner Patentanwälte mbB

(56) References cited:
- WO-A1-2015/033938
- JP-A- H0 948 854
- JP-A- H10 182 823
- US-A- 6 111 143
- EISHUN TSUCHIDA ET AL: "SYNTHESIS OF HIGH MOLECULAR WEIGHT POLY(PHENYLENE SULFIDE) BY OXIDATIVE POLYMERIZATION VIA POLY(SULFONIUM CATION) FROM METHYL PHENYL SULFOXIDE", MACROMOLECULES, AMERICAN CHEMICAL SOCIETY, US, vol. 26, no. 26, 20 December 1993 (1993-12-20), pages 7144-7148, XP000416281, ISSN: 0024-9297, DOI: 10.1021/MA00078A005

## Description

### TECHNICAL FIELD

The disclosure relates to a method for preparing an aromatic sulfide or a salt thereof.

### BACKGROUND

Aromatic sulfides are important reactants in the field of organic synthesis. For example, an aromatic sulfide can serve as important intermediates reactants for preparing pesticides and pharmaceuticals. The main method for preparing aromatic alkyl sulfide (such as thioanisole) includes reacting thiophenol with alkyl chloride (such as methyl chloride) in alkaline conditions. Due to the high cost of thiophenol, the preparation cost of aromatic alkyl sulfide is increased.

WO 2015/033938 A1 relates to a polyarylene sulfide resin manufacturing method comprising a step for obtaining a polyarylene sulfide resin having constituent units by reacting a poly(arylene sulfonium salt), which has constituent units.

Eishun Tschuchida et al., Synthesis of High Molecular Weight Poly(phenylene sulfide) by Oxidative Polymerization via Poly(sulfonium cation) from Methyl Phenyl Sulfoxide, Macromolecules, American Chemical Society, US, vol. 26, no. 26, 20 December 1993, pages 7144-7148, describes the formation of high molecular weight poly(phenylene sulfide) having a Mw of more than 2 x 10⁵ via a poly(sulfonium cation) through oxidative polymerization of methyl 4-(phenylthio)phenyl sulfoxide.

US 6,111,143 relates to a sulfonium salt, including its manufacturing method, which is effectively used as a photoacid initiator or radical photoinitiator during polymerization and a photoacid generator, leaving the protection groups of organic compounds, especially as an useful photoacid generator of the chemically amplified photoresist employed in semiconductor materials.

JP H10 182823 A describes a production process for producing a polythioarylene compound. According to this process, a linear high-molecular-weight polythioperfluorphenylene or the like is described to be obtained in high yields under mild conditions by dealkylating or dearylating a polyarylene sulfonium salt compound.

JP H09 48854 A describes producing a compound which is described to be utilizable to a photoresist as an initiator of photopolymerization of a cationic polymerizable monomer and a photosensitive acid generator by polymerizing a sulfoxide compound or a sulfide compound. This compound is obtained by polymerizing a sulfoxide compound in an acid, oxidative polymerizing a sulfide compound by using an oxidizer or an oxidative polymerization catalyst, or by electrolytic oxidative polymerizing.

Therefore, a novel method for preparing aromatic sulfide is needed.

### SUMMARY

The present invention is defined by the subject-matter of the independent claim, wherein preferred embodiments are described by the dependent claims. According to embodiments of the disclosure, the present invention provides a method for preparing aromatic sulfide or a salt thereof. The method includes reacting a compound having a structure represented by Formula (I) to a compound having a structure represented by Formula (III) in the presence of a compound having a structure represented by Formula (II) to obtain a compound having a structure represented by Formula (IV) 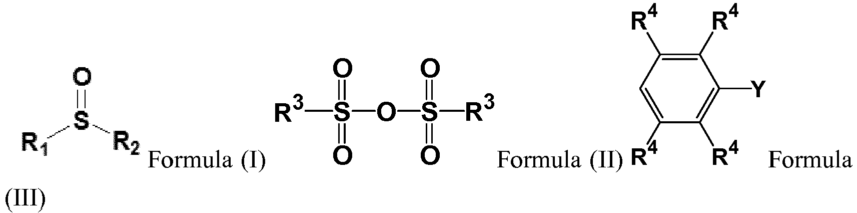 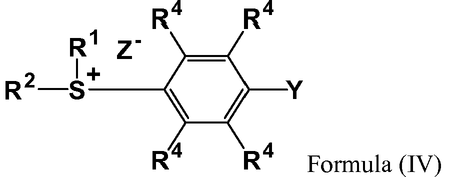 , wherein R¹ and R² are independently C₁₋₆ alkyl group, or C₅₋₇ cycloalkyl group; R³ is independently C₁₋₆ alkyl group, C₅₋₇ cycloalkyl group, C₁₋₆ haloalkyl group, or aryl group; R⁴ is independently hydrogen, or C₁₋₆ alkyl group ; Y is -X-R⁵, wherein X is -O-, -NH-,-PH-, or -S-, , R⁵ is hydrogen, C₁₋₆ alkyl group, C₅₋₇ cycloalkyl group, or aryl group; and Z⁻ is R³SO₃⁻.

It may be provided that R¹ and R² are independently methyl, ethyl, propyl, isopropyl, n-butyl, t-butyl, sec-butyl, isobutyl, pentyl, hexyl, cyclopentyl, cyclohexyl, or cycloheptyl.

It may further be provided that R³ is independently methyl, ethyl, propyl, isopropyl, n-butyl, t-butyl, sec-butyl, isobutyl, pentyl, hexyl, fluoromethyl, fluoroethyl, fluoropropyl, cyclopentyl, cyclohexyl, or cycloheptyl.

It may further be provided that R⁴ is independently hydrogen, methyl, ethyl, propyl, isopropyl, n-butyl, t-butyl, sec-butyl, isobutyl, pentyl, or hexyl.

It may further be provided that the compound having a structure represented by Formula (I) is 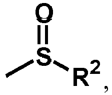 R² is C₁₋₆ alkyl group, or C₅₋₇ cycloalkyl group.

It may further be provided that the compound having a structure represented by Formula (I) is dimethyl sulfoxide, diethyl sulfoxide, or methyl ethyl sulfoxide.

It may further be provided that the compound having a structure represented by Formula (II) is methanesulfonic anhydride, ethanesulfonic anhydride, propanesulfonic anhydride, or trifluoromethanesulfonic anhydride.

It may further be provided that the compound having a structure represented by Formula (III) is 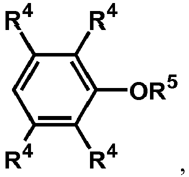 wherein R⁴ is independently hydrogen, or C₁₋₆ alkyl group ; and, R⁵ is hydrogen, C₁₋₆ alkyl group, C₅₋₇ cycloalkyl group, or aryl group.

It may further be provided that reacting the compound having the structure represented by Formula (I) to the compound having the structure represented by Formula (III) in the presence of the compound having the structure represented by Formula (II) comprises reacting the compound having the structure represented by Formula (I) to diphenyl ether in the presence of the compound having the structure represented by Formula (II).

It may further be provided that the compound having a structure represented by Formula (IV) is 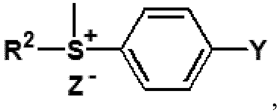 wherein R² is C₁₋₆ alkyl group, or C₅₋₇ cycloalkyl group; Y is -X-R⁵, wherein X is -O-, -NH-, -PH-, or -S-; and R⁵ is H, C₁₋₆ alkyl group, C₅₋₇ cycloalkyl group, or aryl group; and Z⁻ is R³SO₃⁻.

It may further be provided that the method further comprises: reacting a nucleophile with the compound having a structure represented by Formula (IV), obtaining a compound having a structure represented by Formula (V), 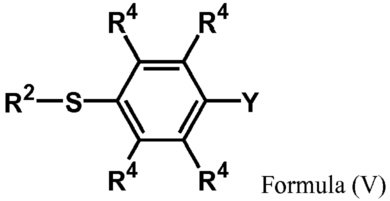 , wherein R² is C₁₋₆ alkyl group, or C₅₋₇ cycloalkyl group; R⁴ is independently hydrogen, or C₁₋₆ alkyl group ; Y is -X-R⁵, wherein X is -O-, -NH-, -PH-, or -S-; and R⁵ is H, C₁₋₆ alkyl group, C₅₋₇ cycloalkyl group, or aryl group.

It may further be provided that the nucleophile is substituted or unsubstituted pyridine or derivatives thereof, amine, halogenated salt, alcohol, or amide.

It may further be provided that the nucleophile is pyridine, 4-methylpyridine, triethylamine, potassium chloride, methanol, ethanol, dimethylformamide, dimethylacetamide, N-methylpyrrolidone, or a combination thereof.

It may further be provided that the compound having a structure represented by Formula (V) is 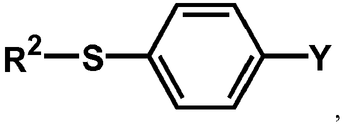 wherein R² is C₁₋₆ alkyl group, or C₅₋₇ cycloalkyl group; Y is -X-R⁵, wherein X is -O-, -NH-, -PH-, or -S-; and R⁵ is H, C₁₋₆ alkyl group, C₅₋₇ cycloalkyl group, or aryl group.

It may further be provided that the compound having a structure represented by Formula (V) is

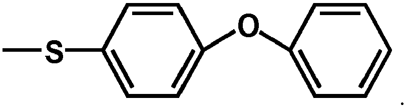

A detailed description is given in the following embodiments with reference to the accompanying drawings.

### DETAILED DESCRIPTION

Embodiments of the disclosure provide a method for preparing aromatic sulfide or a salt thereof. Due to the use of sulfoxide as a vulcanizing agent and the use of sulfonic anhydride for activating the sulfoxide, the method for preparing aromatic sulfide or a salt thereof of the disclosure can be performed without thiophenol serving as a reactant. As a result, the preparation cost of the aromatic sulfide prepared by the method for preparing aromatic sulfide or a salt thereof of the disclosure is reduced and the yield of the aromatic sulfide prepared by the method for preparing aromatic sulfide or a salt thereof of the disclosure is improved, and there is no halogen-containing byproduct remaining in the aromatic sulfide prepared by the method for preparing aromatic sulfide or a salt thereof of the disclosure.

According to embodiments of the disclosure, the method includes reacting a compound having a structure represented by Formula (I) to a compound having a structure represented by Formula (III) in the presence of a compound having a structure represented by Formula (II) to obtain a compound having a structure represented by Formula (IV) 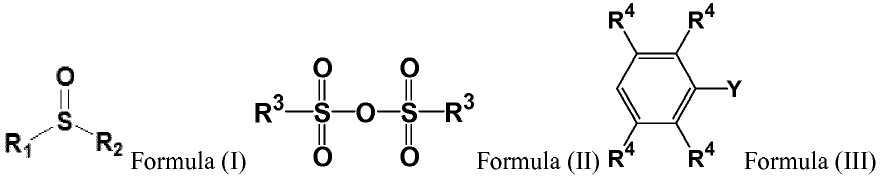 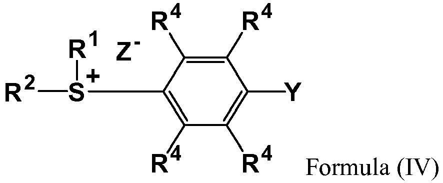 , wherein R¹ and R² are independently C₁₋₆ alkyl group, or C₅₋₇ cycloalkyl group; R³ is independently C₁₋₆ alkyl group, C₅₋₇ cycloalkyl group, C₁₋₆ haloalkyl group, or aryl group; R⁴ is independently hydrogen, or C₁₋₆ alkyl group ; Y is -X-R⁵, wherein X is -O-, -NH-,-PH-, or -S-, , R⁵ is hydrogen, C₁₋₆ alkyl group, C₅₋₇ cycloalkyl group, or aryl group; and Z⁻ is R³SO₃⁻. According to embodiments of the disclosure, the compound having a structure represented by Formula (I) can serve as a vulcanizing agent. Due to the presence of the sulfonic anhydride, the sulfoxide (S=O) functional group can be protonated to form a sulfonium hydroxide functional group with reactivity, and then the sulfonium hydroxide functional group can react with the compound having a structure represented by Formula (III) to undergo an electrophilic substitution reaction to obtain an aromatic sulfide salt. In addition, a nucleophile can be reacted with the aromatic sulfide salt to undergo a dealkylation, obtaining aromatic sulfide.

According to embodiments of the disclosure, the alkyl group can be a linear or branched alkyl group. Therefore, R¹ and R² can be independently methyl, ethyl, propyl, isopropyl, n-butyl, t-butyl, sec-butyl, isobutyl, pentyl, hexyl, cyclopentyl, cyclohexyl, or cycloheptyl. In addition, According to embodiments of the disclosure, the compound having a structure represented by Formula (I) can be 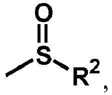 and thus the method of the disclosure can prepare aryl methyl sulfide or a salt thereof, wherein R² has the same definition as above. Moreover, according to embodiments of the disclosure, the compound having a structure represented by Formula (I) can be dimethyl sulfoxide, diethyl sulfoxide, or methyl ethyl sulfoxide.

According to embodiments of the disclosure, the haloalkyl group means that hydrogen atoms bonded to carbon atoms of the alkyl group can be partially or totally replaced with halogen. The haloalkyl group can be a linear or branched haloalkyl group. For example, fluoromethyl can be -CH₂F, -CHF₂- or -CF₃. According to embodiments of the disclosure, R³ can be independently methyl, ethyl, propyl, isopropyl, n-butyl, t-butyl, sec-butyl, isobutyl, pentyl, hexyl, fluoromethyl, fluoroethyl, fluoropropyl, cyclopentyl, cyclohexyl, or cycloheptyl. According to some embodiments of the disclosure, the compound having a structure represented by Formula (II) can be methanesulfonic anhydride, ethanesulfonic anhydride, propanesulfonic anhydride, or trifluoromethanesulfonic anhydride, or a combination thereof. According to embodiments of the disclosure, the molar ratio of the compound having a structure represented by Formula (II) to the compound having a structure represented by Formula (I) can be from about 1 to 2, such as from about 1.1 to 1.5. Herein, due to the sulfonic anhydride, an acidic condition is provided, thereby promoting the reaction of the compound having a structure represented by Formula (I) with the compound having a structure represented by Formula (III). In addition, according to embodiments of the disclosure, the excessive sulfonic anhydride can also serve as a reaction solvent.

According to embodiments of the disclosure, the molar ratio of the compound having a structure represented by Formula (III) to the compound having a structure represented by Formula (I) can be from about 1.2 to 10, such as from about 1.2 to 5. R⁴ can be independently hydrogen, methyl, ethyl, propyl, isopropyl, n-butyl, t-butyl, sec-butyl, isobutyl, pentyl, or hexyl; Y can be hydrogen, phenyl, -O-R⁵, -NH-R⁵, -S-R⁵, or-PH-R⁵; and R⁵ can be hydrogen, methyl, ethyl, propyl, isopropyl, n-butyl, t-butyl, sec-butyl, isobutyl, pentyl, hexyl, cyclopentyl, cyclohexyl, cycloheptyl, or phenyl. According to embodiments of the disclosure, the compound having a structure represented by Formula (III) is 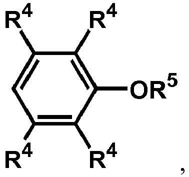 wherein R⁴ and R⁵ have the same definition as above. For example, the compound having a structure represented by Formula (III) can be, diphenyl amine, diphenyl sulfide, or diphenyl ether.

According to embodiments of the disclosure, Y of the compound having a structure represented by Formula (III) and an adjacent R⁴ can be optionally combined with the carbon atoms to which they are attached, to form an aryl group. For example, the compound having a structure represented by Formula (III) can be naphthalene.

According to embodiments of the disclosure, the compound having a structure represented by Formula (IV) can be 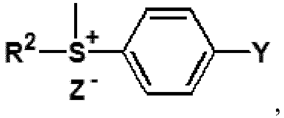 wherein R², Y, and Z⁻ have the same definition as above.

According to embodiments of the disclosure, after obtaining the compound having a structure represented by Formula (IV) (i.e. an aromatic sulfide salt), the method for preparing aromatic sulfide or a salt thereof can further include reacting a nucleophile with the compound having a structure represented by Formula (IV), obtaining a compound having a structure represented by Formula (V) (aromatic sulfide). 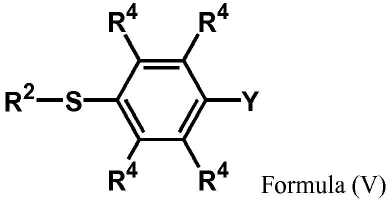 , wherein R² is C₁₋₆ alkyl group, or C₅₋₇ cycloalkyl group; Y is -X-R⁵, wherein X is -O-,-NH-, -PH-, or -S-, and R⁵ is H, C₁₋₆ alkyl group, C₅₋₇ cycloalkyl group, or aryl group.

According to embodiments of the disclosure, the compound having a structure represented by Formula (V) can be 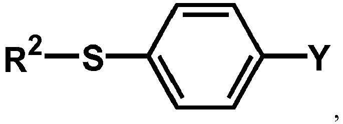 wherein R², and Y have the same definition as above. In addition, the compound having a structure represented by Formula (V) can be

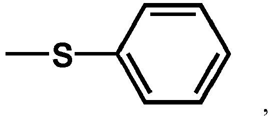

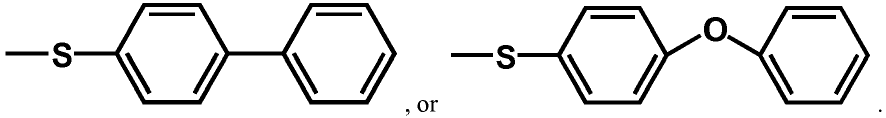

According to embodiments of the disclosure, the nucleophile can be substituted or unsubstituted pyridine or derivatives thereof (such as pyridine or 4-methylpyridine), amine (such as triethylamine), halogenated salt (such as potassium chloride), alcohol (such as methanol or ethanol), or amide (such as dimethylformamide, dimethylacetamide, or N-methylpyrrolidone). The molar ratio of the nucleophile to the compound having a structure represented by Formula (IV) can be from about 1 to 10.

The inventive concept of the disclosure may be embodied in various forms without being limited to the exemplary embodiments set forth herein.

### Example 1 (comparative example):

13ml of benzene (141.5mmol), 2ml of dimethyl sulfoxide (28.3mmol), and 50ml of dichloromethane were added into a reaction bottle, and then cooled to -35°C under a nitrogen atmosphere. After stirring for several minutes, 6ml of trifluoromethanesulfonic anhydride (35mmol) was added into the reaction bottle. After stirring at 0°C (ice bath) for 1hr, a solution with white suspension was obtained. Next, after stirring at room temperature for 12hr, the result was concentrated, obtaining Compound 1 with a yield of about 90%. The synthesis pathway of the above reaction was as follows:

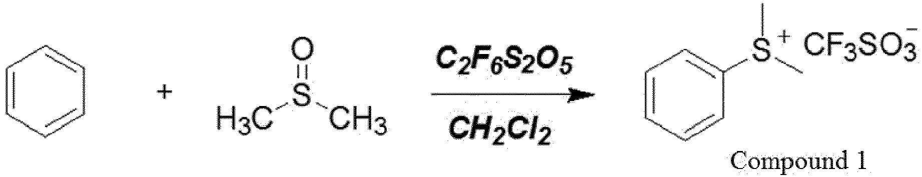

Compound 1 was analyzed by nuclear magnetic resonance (NMR) spectroscopy and the result is as follows: ¹H NMR (400MHz, ppm, Acetone): 3.49 (-CH₃, 6H, s), 8.16-8.18 (phenyl, 2H, d), 7.83-7.86 (phenyl, 1H, t), 7.75-7.79 (phenyl, 2H, t).

Next, 6.52g of Compound 1 (22.6mmol) and 30ml of acetone were added into a reaction bottle. Next, potassium chloride (KCl) aqueous solution (6g of potassium chloride dissolved in 30ml of water) was added slowly into a reaction bottle under a nitrogen atmosphere. After stirring for 1hr, potassium hydroxide (KOH) aqueous solution (4g of potassium hydroxide dissolved in 10ml of water) was added into the reaction bottle. Next, the reaction bottle was heated to reflux (100°C), and the mixture was stirred for 20hr. Next, after cooling, the result was extracted three times using 50ml of water and 50ml of hexane as the extraction solvent, and then the organic phase was collected. After drying, filtering and concentrating the organic phase, methyl phenyl sulfide with a yield of about 87% was obtained. The synthesis pathway of the above reaction was as follows:

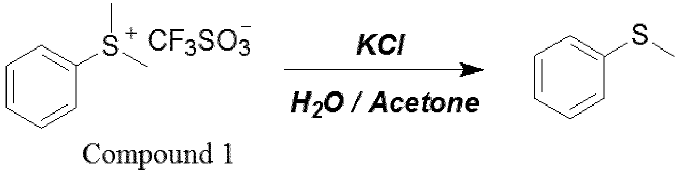

Methyl phenyl sulfide was analyzed by nuclear magnetic resonance (NMR) spectroscopy and the result is as follows: ¹H NMR (400MHz, ppm, CDCl₃): 2.52 (-CH₃, 3H, s), 7.14-7.19 (phenyl, 1H, t), 7.31 (phenyl, 4H, m).

### Example 2 (inventive example):

2.4g of diphenyl ether (14mmol), 0.92g of dimethyl sulfoxide (12mmol), and 15ml of dichloromethane were added into a reaction bottle, and then cooled to -35°C under a nitrogen atmosphere. After stirring for several minutes, 2.3ml of trifluoromethanesulfonic anhydride (14mmol) was added into the reaction bottle. After stirring at 0°C (ice bath) for 1hr, a solution with white suspension was obtained. After stirring at room temperature for 12hr, the result was concentrated and recrystallized with ethyl ether, obtaining Compound 2 with a yield of about 65%. The synthesis pathway of the above reaction was as follows:

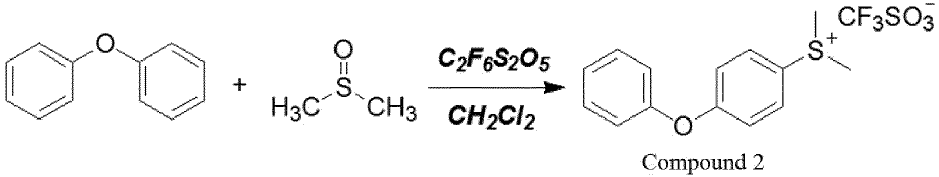

Methyl phenyl sulfide was analyzed by nuclear magnetic resonance (NMR) spectroscopy and the result is as follows: ¹H NMR (400MHz, ppm, Acetone): 3.50 (-CH₃, 6H, s), 8.18 (phenyl, 2H, d), 7.52 (phenyl, 2H, t), 7.26-7.34 (phenyl, 3H, m), 7.18 (phenyl, 2H, d).

Next, 2.9g of Compound 2 (7.6mmol) and 10ml of acetonitrile were added into a reaction bottle. Next, potassium chloride (KCl) aqueous solution (9g of potassium chloride dissolved in 20ml of water) was added slowly into the reaction bottle under a nitrogen atmosphere. After stirring for 1hr, 0.22g of KOH aqueous solution was added into the reaction bottle. Next, the reaction bottle was heated to reflux (100°C), and the mixture was stirred for 17hr. Next, after cooling, the result was extracted three times using 50ml of water and 50ml of hexane as the extraction solvent, and then the organic phase was collected. After drying, filtering and concentrating the organic phase, methyl(4-phenyloxy)phenyl sulfide with a yield of about 60% was obtained. The synthesis pathway of the above reaction was as follows:

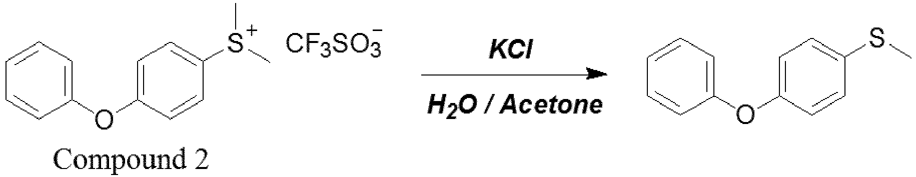

Compound 2 was analyzed by nuclear magnetic resonance (NMR) spectroscopy and the result is as follows: ¹H NMR (400MHz, ppm, CDCl₃): 2.50 (-CH₃, 3H, s), 6.97-7.03 (phenyl, 4H, m), 7.15 (phenyl, 1H, t), 7.32-7.42 (phenyl, 4H, m).

## Claims

1. A method for preparing an aromatic sulfide or a salt thereof, comprising:
reacting a compound having a structure represented by Formula (I) to a compound having a structure represented by Formula (III) in the presence of a compound having a structure represented by Formula (II) to obtain a compound having a structure represented by Formula (IV) 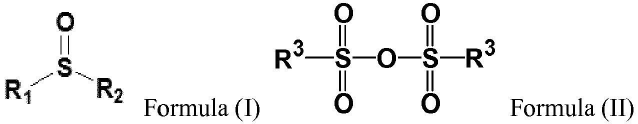 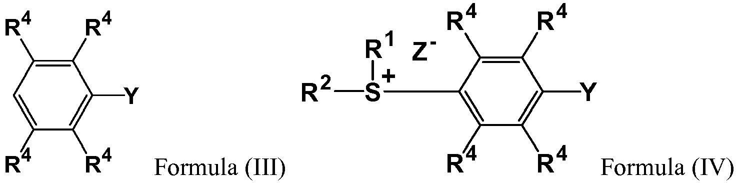
, wherein R¹ and R² are independently C₁₋₆ alkyl group, or C₅₋₇ cycloalkyl group;
R³ is independently C₁₋₆ alkyl group, C₅₋₇ cycloalkyl group, C₁₋₆ haloalkyl group, or aryl group; R⁴ is independently H, or C₁₋₆ alkyl group; Y is -X-R⁵; X is -O-, -NH-, -PH-, or -S-; R⁵ is H, C₁₋₆ alkyl group, C₅₋₇ cycloalkyl group, or aryl group; and, Z⁻ is R³SO₃⁻.

2. The method as claimed in Claim 1, wherein R¹ and R² are independently methyl, ethyl, propyl, isopropyl, n-butyl, t-butyl, sec-butyl, isobutyl, pentyl, hexyl, cyclopentyl, cyclohexyl, or cycloheptyl.

3. The method as claimed in Claim 1, wherein R³ is independently methyl, ethyl, propyl, isopropyl, n-butyl, t-butyl, sec-butyl, isobutyl, pentyl, hexyl, fluoromethyl, fluoroethyl, fluoropropyl, cyclopentyl, cyclohexyl, or cycloheptyl.

4. The method as claimed in Claim 1, wherein R⁴ is independently hydrogen, methyl, ethyl, propyl, isopropyl, n-butyl, t-butyl, sec-butyl, isobutyl, pentyl, or hexyl.

5. The method as claimed in Claim 1, wherein the compound having a structure represented by Formula (I) is 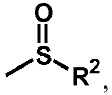 R² is C₁₋₆ alkyl group, or C₅₋₇ cycloalkyl group.

6. The method as claimed in Claim 1, wherein the compound having a structure represented by Formula (I) is dimethyl sulfoxide, diethyl sulfoxide, or methyl ethyl sulfoxide.

7. The method as claimed in Claim 1, wherein the compound having a structure represented by Formula (II) is methanesulfonic anhydride, ethanesulfonic anhydride, propanesulfonic anhydride, or trifluoromethanesulfonic anhydride.

8. The method as claimed in Claim 1, wherein the compound having a structure represented by Formula (III) is 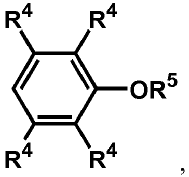 wherein R⁴ is independently hydrogen, or C₁₋₆ alkyl group ; and, R⁵ is hydrogen, C₁₋₆ alkyl group, C₅₋₇ cycloalkyl group, or aryl group.

9. The method as claimed in Claim 1, wherein reacting the compound having the structure represented by Formula (I) to the compound having the structure represented by Formula (III) in the presence of the compound having the structure represented by Formula (II) comprises reacting the compound having the structure represented by Formula (I) to diphenyl ether in the presence of the compound having the structure represented by Formula (II).

10. The method as claimed in Claim 1, wherein the compound having a structure represented by Formula (IV) is 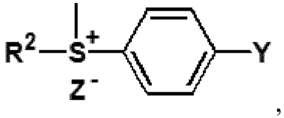 wherein R² is C₁₋₆ alkyl group, or C₅₋₇ cycloalkyl group; Y is -X-R⁵, wherein X is -O-, -NH-, -PH-, or -S-; and R⁵ is H, C₁₋₆ alkyl group, C₅₋₇ cycloalkyl group, or aryl group; and Z⁻ is R³SO₃⁻.

11. The method as claimed in Claim 1, further comprising:
reacting a nucleophile with the compound having a structure represented by Formula (IV), obtaining a compound having a structure represented by Formula (V), 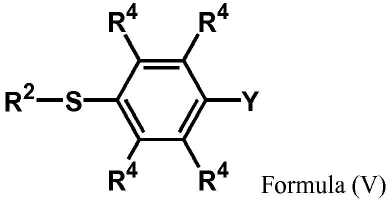
, wherein R² is C₁₋₆ alkyl group, or C₅₋₇ cycloalkyl group; R⁴ is independently hydrogen, or C₁₋₆ alkyl group ; Y is -X-R⁵, wherein X is -O-, -NH-, -PH-, or -S-; and
R⁵ is H, C₁₋₆ alkyl group, C₅₋₇ cycloalkyl group, or aryl group.

12. The method as claimed in Claim 11, wherein the nucleophile is substituted or unsubstituted pyridine or derivatives thereof, amine, halogenated salt, alcohol, or amide.

13. The method as claimed in Claim 11, wherein the nucleophile is pyridine, 4-methylpyridine, triethylamine, potassium chloride, methanol, ethanol, dimethylformamide, dimethylacetamide, N-methylpyrrolidone, or a combination thereof.

14. The method as claimed in Claim 11, wherein the compound having a structure represented by Formula (V) is 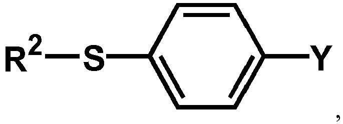 wherein R² is C₁₋₆ alkyl group, or C₅₋₇ cycloalkyl group; Y is -X-R⁵, wherein X is -O-, -NH-, -PH-, or -S-; and R⁵ is H, C₁₋₆ alkyl group, C₅₋₇ cycloalkyl group, or aryl group.

15. The method as claimed in Claim 11, wherein the compound having a structure represented by Formula (V) is

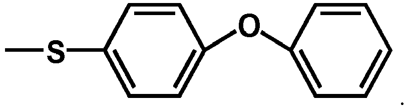

## Patentansprüche

1. Verfahren zum Herstellen eines aromatischen Sulfids oder eines Salzes davon, mit den Schritten:
Umsetzen einer Verbindung mit einer durch Formel (I) dargestellten Struktur mit einer Verbindung mit einer durch Formel (III) dargestellten Struktur in Gegenwart einer Verbindung mit einer durch Formel (II) dargestellten Struktur, um eine Verbindung mit einer durch Formel (IV) dargestellten Struktur zu erhalten 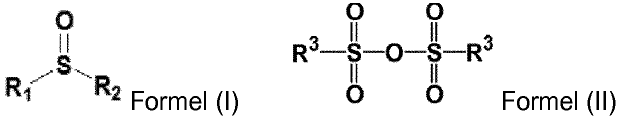 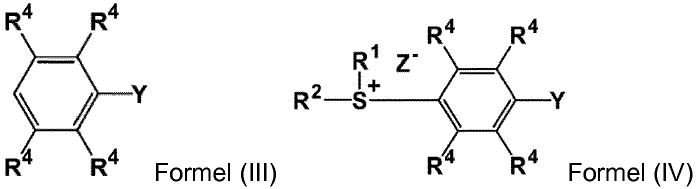 wobei R¹ und R² unabhängig voneinander eine C₁₋₆-Alkylgruppe oder eine C₅₋₇-Cycloalkylgruppe sind, R³ unabhängig eine C₁₋₆-Alkylgruppe, eine C₅₋₇-Cycloalkylgruppe, eine C₁₋₆-Haloalkylgruppe oder Arylgruppe ist, R⁴ unabhängig H oder eine C₁₋₆-Alkylgruppe ist, Y-X-R⁵ ist, X -O-, -NH-, -PH- oder -S- ist, R⁵ H, eine C₁₋₆-Alkylgruppe, eine C₅₋₇-Cycloalkylgruppe oder eine Arylgruppe ist und Z⁻ R³SO₃⁻ ist.

2. Verfahren nach Anspruch 1, wobei R¹ und R² unabhängig voneinander Methyl, Ethyl, Propyl, Isopropyl, n-Butyl, t-Butyl, sec-Butyl, Isobutyl, Pentyl, Hexyl, Cyclopentyl, Cyclohexyl oder Cycloheptyl sind.

3. Verfahren nach Anspruch 1, wobei R³ unabhängig Methyl, Ethyl, Propyl, Isopropyl, n-Butyl, t-Butyl, sec-Butyl, Isobutyl, Pentyl, Hexyl, Fluormethyl, Fluorethyl, Fluorpropyl, Cyclopentyl, Cyclohexyl oder Cycloheptyl ist.

4. Verfahren nach Anspruch 1, wobei R⁴ unabhängig Wasserstoff, Methyl, Ethyl, Propyl, Isopropyl, n-Butyl, t-Butyl, sec-Butyl, Isobutyl, Pentyl oder Hexyl ist.

5. Verfahren nach Anspruch 1, wobei die Verbindung, die eine durch Formel (I) dargestellte Struktur hat, 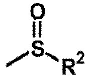 ist, wobei R² eine C₁₋₆-Alkylgruppe oder eine C₅₋₇-Cycloalkylgruppe ist.

6. Verfahren nach Anspruch 1, wobei die Verbindung, die eine durch Formel (I) dargestellte Struktur hat, Dimethylsulfoxid, Diethylsulfoxid oder Methylethylsulfoxid ist.

7. Verfahren nach Anspruch 1, wobei die Verbindung, die eine durch Formel (II) dargestellte Struktur hat, Methansulfonsäureanhydrid, Ethansulfonsäureanhydrid, Propansulfonsäureanhydrid oder Trifluormethansulfonsäureanhydrid ist.

8. Verfahren nach Anspruch 1, wobei die Verbindung, die eine durch Formel (III) dargestellte Struktur hat, 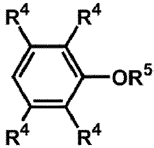 ist, wobei R⁴ unabhängig Wasserstoff oder eine C₁₋₆-Alkylgruppe ist und R⁵ Wasserstoff, eine C₁₋₆-Alkylgruppe, eine C₅₋₇-Cycloalkylgruppe oder eine Arylgruppe ist.

9. Verfahren nach Anspruch 1, wobei das Umsetzen der Verbindung mit der durch Formel (I) dargestellten Struktur mit der Verbindung mit der durch Formel (III) dargestellten Struktur in Gegenwart der Verbindung mit der durch Formel (II) dargestellten Struktur das Umsetzen der Verbindung mit der durch Formel (I) dargestellten Struktur mit Diphenylether in Gegenwart der Verbindung mit der durch Formel (II) dargestellten Struktur aufweist.

10. Verfahren nach Anspruch 1, wobei die Verbindung mit einer durch Formel (IV) dargestellten Struktur 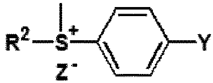 ist, wobei R² eine C₁₋₆-Alkylgruppe oder eine C₅₋₇-Cycloalkylgruppe ist, Y-X-R⁵ ist, wobei X -O-, -NH-,-PH- oder -S- ist, R⁵ H, eine C1-6-Alkylgruppe, eine C₅₋₇-Cycloalkylgruppe oder eine Arylgruppe ist, und wobei T R³SO₃⁻ ist.

11. Verfahren nach Anspruch 1, ferner mit dem Umsetzen eines Nucleophils mit der Verbindung mit einer durch Formel (IV) dargestellten Struktur, um eine Verbindung mit einer durch Formel (V) dargestellten Struktur zu erhalten, 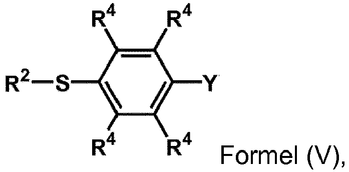 wobei R² eine C₁₋₆-Alkylgruppe oder eine C₅₋₇-Cycloalkylgruppe ist, R⁴ unabhängig Wasserstoff oder eine C₁₋₆-Alkylgruppe ist, Y-X-R⁵ ist, wobei X -O-, -NH-, -PH- oder -S- ist, und R⁵ H, eine C₁₋₆-Alkylgruppe, eine C₅₋₇-Cycloalkylgruppe oder eine Arylgruppe ist.

12. Verfahren nach Anspruch 11, wobei das Nucleophil substituiertes oder unsubstituiertes Pyridin oder Derivate davon, Amin, halogeniertes Salz, Alkohol oder Amid ist.

13. Verfahren nach Anspruch 11, wobei das Nucleophil Pyridin, 4-Methylpyridin, Triethylamin, Kaliumchlorid, Methanol, Ethanol, Dimethylformamid, Dimethylacetamid, N-Methylpyrrolidon oder eine Kombination davon ist.

14. Verfahren nach Anspruch 11, wobei die Verbindung, die eine durch Formel (V) dargestellte Struktur hat, 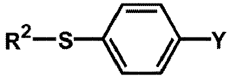 ist, wobei R² eine C₁₋₆-Alkylgruppe oder eine C₅₋₇-Cycloalkylgruppe ist, Y-X-R⁵ ist, wobei X -O-, -NH-, -PH- oder -S- ist, und R⁵ H, eine C₁₋₆-Alkylgruppe, eine C₅₋₇-Cycloalkylgruppe oder eine Arylgruppe ist.

15. Verfahren nach Anspruch 11, wobei die Verbindung, die eine durch die Formel (V) dargestellte Struktur hat, 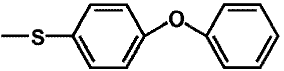 ist.

## Revendications

1. Procédé de préparation d'un sulfure aromatique ou d'un sel de celui-ci, comprenant :
la réaction d'un composé ayant une structure représentée par la formule (I) avec un composé ayant une structure représentée par la formule (III) en présence d'un composé ayant une structure représentée par la formule (II) pour obtenir un composé ayant une structure représentée par la formule (IV) 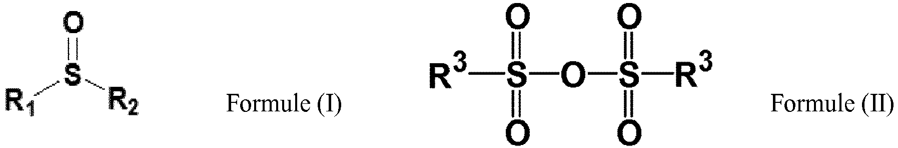 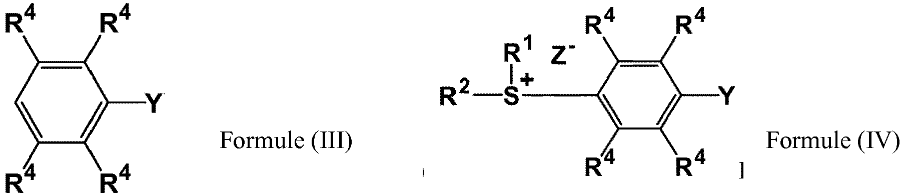 où R¹ et R² sont de manière indépendante un groupe alkyle en C₁₋₆ ou un groupe cycloalkyle en C₅₋₇ ; R³ est de manière indépendante un groupe alkyle en C₁₋₆, un groupe cycloalkyle en C₅₋₇, un groupe haloalkyle en C₁₋₆ ou un groupe aryle ; R⁴ est de manière indépendante un atome H ou un groupe alkyle en C₁₋₆ ; Y équivaut à -X-R⁵ ; X équivaut à -O-, -NH-, -PH- ou à -S- ; R⁵ est un atome H, un groupe alkyle en C₁₋₆, un groupe cycloalkyle en C₅₋₇, ou un groupe aryle ; et Z⁻ équivaut à R³SO₃⁻.

2. Procédé selon la revendication 1, dans lequel R¹ et R² sont de manière indépendante des groupes méthyle, éthyle, propyle, isopropyle, n-butyle, t-butyle, butyle secondaire, isobutyle, pentyle, hexyle, cyclopentyle, cyclohexyle ou cycloheptyle.

3. Procédé selon la revendication 1, dans lequel R³ est de manière indépendante un groupe méthyle, éthyle, propyle, isopropyle, n-butyle, t-butyle, butyle secondaire, isobutyle, pentyle, hexyle, fluorométhyle, fluorométhyle, fluoropropyle, cyclopentyle, cyclohexyle ou cycloheptyle.

4. Procédé selon la revendication 1, dans lequel R⁴ est de manière indépendante un atome d'hydrogène, un groupe méthyle, éthyle, propyle, isopropyle, n-butyle, t-butyle, butyle secondaire, isobutyle, pentyle ou hexyle.

5. Procédé selon la revendication 1, dans lequel le composé ayant une structure représentée par la formule (I) est un groupe 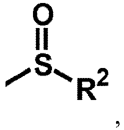 R² est un groupe alkyle en C₁₋₆ ou un groupe cycloalkyle en C₅₋₇.

6. Procédé selon la revendication 1, dans lequel le composé ayant une structure représentée par la formule (I) est du diméthyl sulfoxyde, du diéthyl sulfoxyde, ou du méthyl éthyl sulfoxyde.

7. Procédé selon la revendication 1, dans lequel le composé ayant une structure représentée par la formule (II) est de l'anhydride méthane sulfonique, de l'anhydride éthane sulfonique, de l'anhydride propane sulfonique ou de l'anhydride trifluoro méthane sulfonique.

8. Procédé selon la revendication 1, dans lequel le composé ayant une structure représentée par la formule (III) est 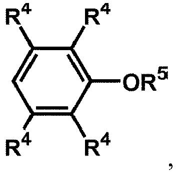 où R⁴ est de manière indépendante un atome d'hydrogène ou un groupe alkyle en C₁₋₆ ; et R⁵ est un atome d'hydrogène, un groupe alkyle en C₁₋₆, un groupe cycloalkyle en C₅₋₇ ou un groupe aryle.

9. Procédé selon la revendication 1, dans lequel la réaction du composé ayant une structure représentée par la formule (I) avec le composé ayant la structure représentée par la formule (III) en présence du composé ayant la structure représentée par la formule (II) comprend la réaction du composé ayant la structure représentée par la formule (I) avec du diphényl éther en présence du composé ayant la structure représentée par la formule (II).

10. Procédé selon la revendication 1, dans lequel le composé ayant une structure représentée par la formule (IV) est 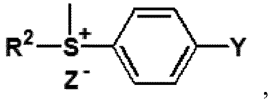 où R² est un groupe alkyle en C₁₋₆ ou un groupe cycloalkyle en C₅₋₇ ; Y équivaut à -X-R⁵, où X équivaut à -O-, -NH-, -PH- ou à -S- ; R⁵ est un atome H, un groupe alkyle en C₁₋₆, un groupe cycloalkyle en C₅₋₇ ou un groupe aryle ; et Z⁻ équivaut à R³SO₃⁻.

11. Procédé selon la revendication 1, comprenant en outre :
la réaction d'un nucléophile avec le composé ayant une structure représentée par la formule (IV), en obtenant un composé ayant une structure représentée par la formule (V) 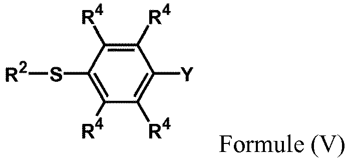 où R² est un groupe alkyle en C₁₋₆ ou un groupe cycloalkyle en C₅₋₇ ; R⁴ est de manière indépendante un atome d'hydrogène ou un groupe alkyle en C₁₋₆ ; Y équivaut à -X-R⁵, où X équivaut à -O-, -NH-, -PH- ou à -S- ; et R⁵ est un atome H, un groupe alkyle en C₁₋₆, un groupe cycloalkyle en C₅₋₇ ou un groupe aryle.

12. Procédé selon la revendication 11, dans lequel le nucléophile est de la pyridine substituée ou non substituée ou des dérivés de celle-ci, une amine, un sel halogéné, un alcool ou un amide.

13. Procédé selon la revendication 11, dans lequel le nucléophile est la pyridine, la 4-méthylpyridine, la triéthylamine, le chlorure de potassium, le méthanol, l'éthanol, le diméthylformamide, le diméthylacétamide, la N-méthylpyrrolidone ou une combinaison de ceux-ci.

14. Procédé selon la revendication 11, dans lequel le composé ayant une structure représentée par la formule (V) est 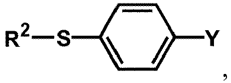 où R² est un groupe alkyle en C₁₋₆ ou un groupe cycloalkyle en C₅₋7 ; Y équivaut à -X-R⁵, où X équivaut à -O-, -NH-, -PH- ou à -S- ; et R⁵ est un atome H, un groupe alkyle en C₁₋₆, un groupe cycloalkyle en C₅₋₇ ou un groupe aryle.

15. Procédé selon la revendication 11, dans lequel le composé ayant une structure représentée par la formule (V) est
